# EUROPEAN PATENT SPECIFICATION

(11) **EP 3 501 452 B1**
(45) Date of publication and mention of the grant of the patent: **21.02.2024**
(21) Application number: 18191986.1
(22) Date of filing: 31.08.2018
(51) Int. Cl.: A61F 2/08, A61B 17/86

(54) **MEDICAL IMPLANT**
MEDIZINISCHES IMPLANTAT
IMPLANT MEDICALE

(30) Priority: 31.10.2017 PL 42333517
(43) Date of publication of application: 26.06.2019
(73) Proprietor: Krzysztof FICEK - Indywidualna Specjalistyczna Praktyka Lekarska, 43-150 Bierun (PL)
(72) Inventor: FICEK, Krzysztof, 43-150 Bieru (PL); WYLEZOL , Marek, 41-803 Zabrze (PL); MUZALEWSKA, Malgorzata, 43-100 Tychy (PL)
(74) Representative: Malcherek, Piotr

(56) References cited:
- EP-A1- 1 093 773
- FR-A1- 2 802 083
- US-A1- 2006 100 627

## Description

The invention relates to a medical implant, in particular an implant that is made of bioresorbable material and provided for intensification of healing of transplants into parent tissue after surgeries associated with reconstruction of ligaments in tunnel sockets of bones.

Patent description PL 217967 discloses a medical implant that is made of bioresorbable material and provided for healing of transplants after reconstruction of ligaments in tunnel sockets of bones. The medical implant has a form of a tube with a axial throughout conduit whilst the tube wall has a porous structure and comprises throughout orifices extending between the conduit and the outer surface of the tube. Such a medical implant can be inserted into a tunnel socket of bone with the ligament transplant which is inserted through its conduit, whilst the porous structure of the implant wall combined with throughout orifices enable optimum perfusion of blood and other body fluids.

In turn, patent description FR 2802083 discloses an implant that has a substantially cylindrical form and can be made for instance of titanium and coated with an outer film of calcium hydroxyapatite. The implant has outer walls with an internal cavity in between with an outlet provided at one side of the implant whilst the other side is provided with a base with an axial orifice on its far end. The implant has two longitudinal cuts cut in its outer walls and splitting the implant body into separate sections with some axial flexibility. In turn, the outer surface of the implant walls has notches and crests that make up an outer thread.

In addition patent description GB 2417536 discloses a bioresorbable screw designed to fix implants to bones during orthopedic surgeries. The screw has a shank with a male thread, where the outer surface of the screw shank can be rough whilst the shank itself can be hollow. In addition, the outer surface of the shank can be provided with indents or throughout boreholes extending from the outer surface of the screw shank up to its hollowed interior.

US 2006/100627 discloses a fixation device for securing tissue to a bone. The fixation device includes an anchor having a hollow body defining a longitudinal passage, and a plug configured to be received in at least a portion of the passage. The body comprises a cylindrical portion and a tapered tip portion. The cylindrical portion comprises a plurality of thin-walled window covers such that after implantation the window covers are resorbed first relative to other portions of the cylindrical portion for defining a plurality of apertures on the cylindrical portion.

The medical implant according to the present invention has a body in tubular form with a longitudinal axial throughout conduit whilst the body wall has throughout orifices and the outer surface of the body is provided with a protruding crest that forms a screw line serving as a screw thread, where the envelope of the screw line is a straight line. The essence of the invention consists in the fact that a part of the implant length has a thread with its envelope curve that is convergent towards one end of the implant body and has the form of a parametric B-spline curve whilst tangency of that curved section to the straight section of the thread envelope is maintained at the contact point between these two sections. The throughout orifices made in the wall of the implant body are spaced in every pitch of the thread in the bottom of the thread root. Cross-sections of the orifices made throughout the body walls converge towards the direction of the axial conduit in the implant body, in particular the orifices have the form of a truncated cone.

Preferably, the thread envelope converges towards one end of the implant and the converges section makes from 40 to 60% of the total length of the implant, most preferably 50% of length of the implant.

The preferred embodiment of the invention assumes that the implant body has at least two longitudinal cuts cut in the body walls in a portion of the body length at its other end.

If so, it is desirable that these longitudinal cuts are cut down the length portion of no more than 60% of the total implant length, preferably no more than 50%.

The medical implant according to the presented invention enable its safe and controllable insertion into a bone tunnel socket owing to its gradually vanishing thread with the thread envelope in the form of a parametrical B-splined curve. Such a form of the thread enables gradual screwing of the implant into a bone tissue, where the load force of the thread increases in proportion to the screwing depth and reliable axial fixation of the implant in the bone tunnel socket is guaranteed. In addition, the crest that makes a screw thread also reinforces the implant stiffness, which compensates its weakening caused by orifices made in the implant body walls. These orifices that converge towards the axial conduit of the implant body are made in the bottom of the thread root and contribute, together with cutting or disruption of bone tissue while the implant is being screwed into the bone tunnel socket, to intensification of blood and other body fluids penetration into the implant interior, which is beneficial to healing efficiency and quickness of ligaments to be reconstructed. In addition, the most preferred embodiment of the invention comprises longitudinal cuts at one side of the implant walls, which enables deflection of implant walls outwardly after the mounting screw is tightened and the implant is additionally fixed in the bone socket. The said cuts can be also used for introduction of an applicator tool with outer ribs engaged with these cuts and make the implant stiffer during its fixing by screwing.

The invention is disclosed in details in the following embodiments thereof and on relevant drawings, where:
Fig. 1 is a side view of the implant;
Fig. 2 is a perspective view of the implant;
Fig. 3 is a front view of the implant;
Fig. 4 is a side view of the implant according to another embodiment of the present invention;
Fig. 5 and fig. 6 are various perspective views of the implant from fig. 4, and
Fig. 7 is a front view of the implant from fig. 4.

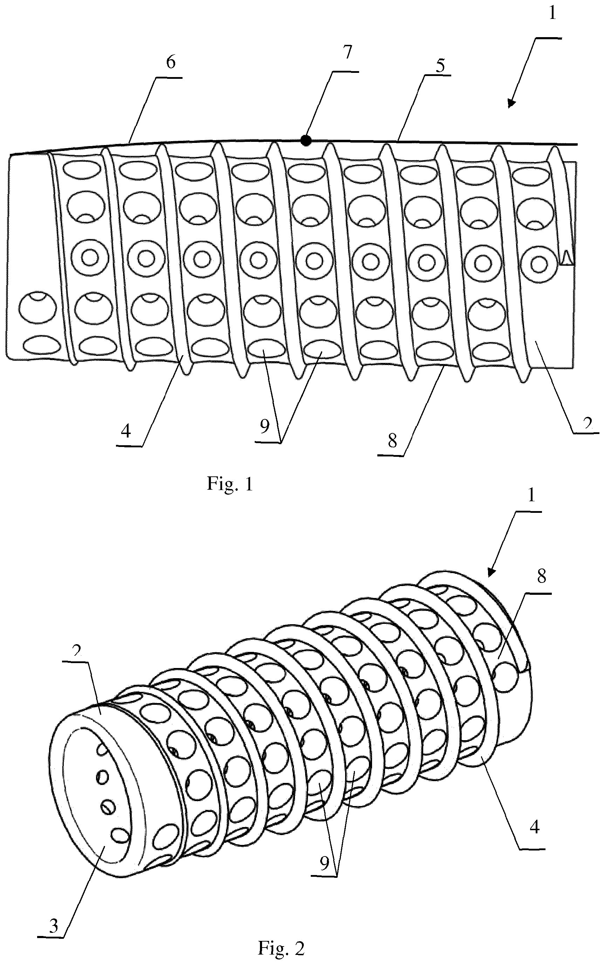

A medical implant 1 as disclosed in fig. 1 to fig. 3 is provided for insertion into a tunnel socket already made in a femoral bone to enable easy healing of transplants after surgeries associated with reconstruction of ligaments. The implant 1 has a body 2 in tubular form with a longitudinal axial throughout conduit 3. The implant 1 is made of bioresorbable material, for instance any material from the group of polyactides. The conduit 3 has an oval or elliptical cross-section, which makes it possible to achieve a desirable position of the transplanted tendon bunch in the conduit 3 and to introduce an applicator tool therein to screw the implant into the bone tunnel socket. Preferably, edges of the inlets into the conduit 3 are rounded, which diminishes the risk to have the tendon transplant damaged while it is inserted via the conduit. The outer surface of the implant body 2 has a protruding crest that forms a screw line serving as a screw thread 4 with a fixed pitch. The envelope of the thread 4, in other words the line that is tangent to the crests of the thread 4 in a planar projection, is a straight line 5 down a specific portion of the implant 1 length. However, the remaining portion of the implant 1 length has a thread 4 with an envelope converging toward one end of the implant 1 and having the shape of a parametrical B-splined curve 6 whilst tangency of that curved sections to the straight section 5 of the thread envelope is maintained at the contact point 7 between these two sections. In particular the thread 4 envelope converges towards one end of the implant 1 down the section that makes from 40 to 60% of the total length of the implant 1, where the 50% ratio of the converging part is the most preferred. For example the total length of the implant 1 is 30 mm whilst the length of the straight-lined envelope is 15 mm and the length of the envelope with the shape of a parametrical B-splined curve is 15 mm. The thread 4 enables axial anchoring of the implant inside a tunnel socket of a bone with potential feasibility to undo the implant when its withdrawal is needed after unsuccessful implantation. In addition, the thread contributes to strengthening of the implant stiffness and vanishing of the thread down the envelope curve enables gradual engagement of the implant material into the bone tissue. Throughout orifices 9 made in the wall of the implant body 2 are spaced in every pitch of the thread 4 in the bottom 8 of the thread root. Cross-section of said orifices 9 is convergent towards the axial conduit 3, in particular the orifices have a form of a truncated cone or similar. Throughout orifices 9 enable perfusion of blood and other body fluids into the tendon bunch being transplanted. Orifices 9 are regularly spaced down the whole screw line and made in the bottom 8 of the thread 4 root. Symmetry axes of said orifices 9 are normal to the screw line as drawn in points where said orifices are made. These converging orifices 9 enable faster and more intense penetration of blood and other body fluids into the implant with simultaneous reduction of the active inner surface of the conduit 3. In addition, the surface of the conduit 3 is smoother since the cross-section areas of orifices is less at their outlets into the conduit 3 Preferably, the orifice outlets at the side of the central conduit are also rounded and smoothed, which leads to less friction when the transplanted tendon is inserted throughout.

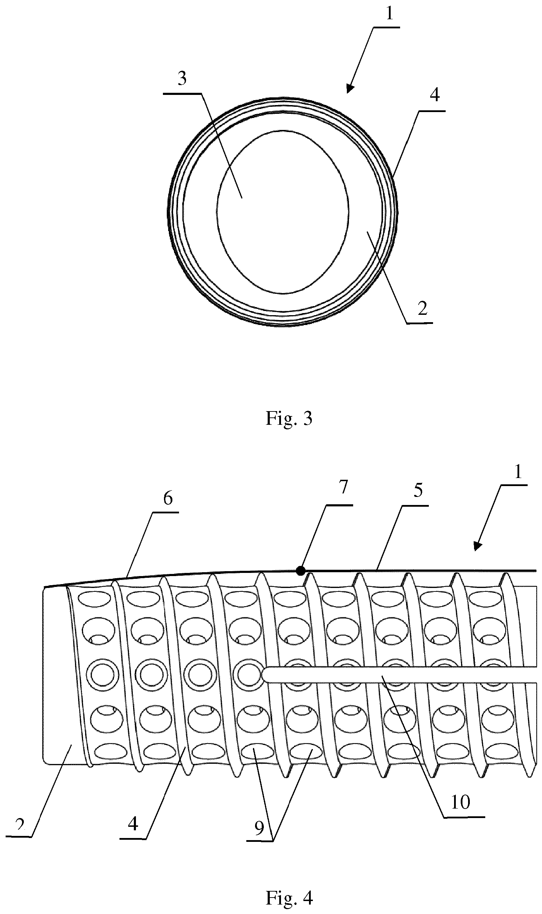

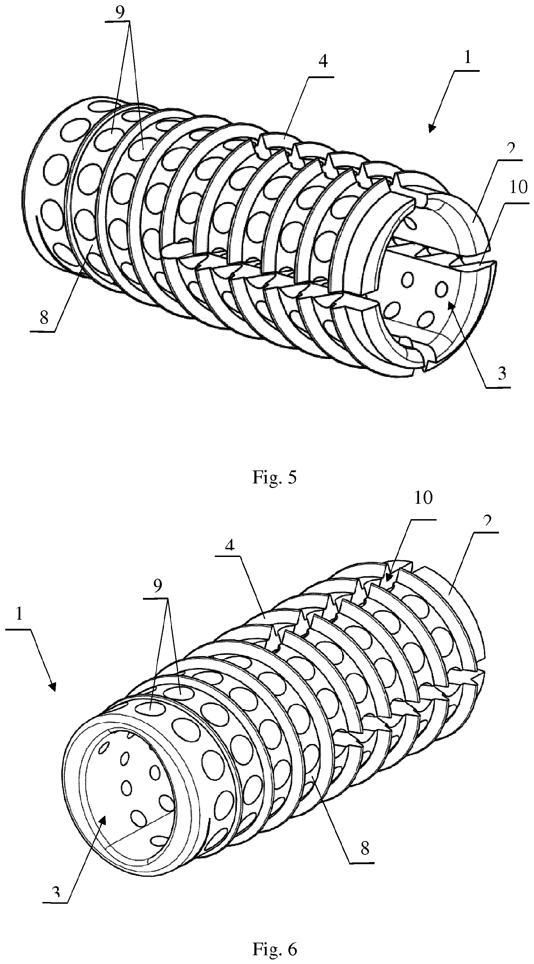

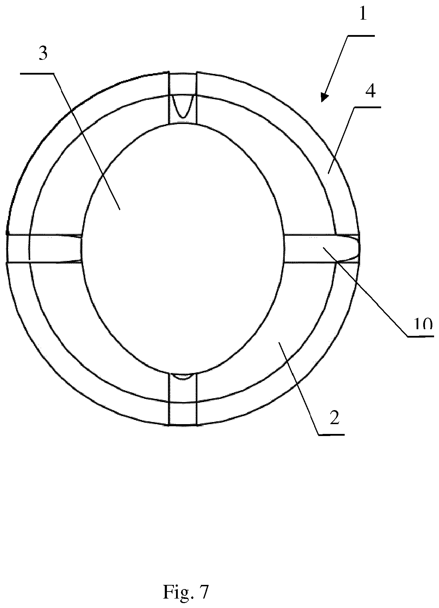

Fig. 4 to fig. 7 disclose another embodiment of the implant 1, in particular an implant designed for a tibia bone. On the contrary to the first embodiment, a portion of the implant 1 length, starting from its far end opposite to the portion with vanishing thread 4, has at least two longitudinal cuts 10 cut in the body 2 walls. The cuts are cut down the section that makes no more than 60% of the total length of the implant 1, where the 50% portion at the most of the cut length is the most preferred embodiment. These cuts 10 are designed for controllable outward deflection of the cut section while a bone wedge is inserted into the implant conduit to have the transplanted tendon fixed inside the implant conduit. In consequence, the implant is more reliably anchored in a tunnel socket of a bone. Said cuts are also useful when the implant is rotated and fixed by means of an applicator tool. The number of cuts 10 can be more than two, for example three or four but it is important to have them symmetrically arranged on the perimeter of the body 2 of the implant 1.

## Claims

1. A medical implant (1), made of bioresorbable material, provided for intensification of healing of transplants into parent tissue after surgeries associated with reconstruction of ligaments in tunnel sockets of bones, where an implant body (2) is a tube with a longitudinal throughout conduit (3), whilst throughout orifices (9) are made in the body (2) wall, and the outer surface of the body (2) is provided with a protruding crest that forms a screw line serving as a screw thread (4), where the envelope of the screw line is a straight line (5), and a part of the implant length has a thread (4) with its envelope curve convergent towards one end of the implant (1) which envelope has the form of a parametric B-spline curve (6), whilst tangency of that curved sections to the straight section (5) of the thread envelope is maintained at the contact point (7) between these two sections, and the throughout orifices (9) made in the wall of the implant body (2) are spaced in every pitch of the thread (4) in the bottom (8) of the thread root, **characterized in that** orifices (9) made in wall of the implant body (2) have cross-sections converging towards the axial conduit (3) of the implant body (2), in particular the orifices have a form of truncated cones.

2. The implant according to claim 1, **characterized in that** the envelope of the thread (4) converges towards one end of the implant (1) down the section with the length from 40% to 60% of the total implant (1) length, most preferably 50% portion of the total implant (1) length.

3. The implant according to claim 1 or 2, **characterized in that** the wall of the implant body (2) has at least two longitudinal cuts (10) that are cut down in a portion of the implant (1) length at its other end.

4. The implant according to claim 3, **characterized in that** cuts (10) are made down the portion of not more than 60% of the total implant (1) length, most preferably not more than 50%.

## Patentansprüche

1. Medizinisches Implantat (1) aus bioresorbierbarem Material zur Verbesserung der Heilung von Transplantaten bei der Rekonstruktion von Bändern in Knochentunneln, dessen Körper (2) die Form einer Röhre mit einem längsaxialen Durchgangskanal (3) hat, während sich in der Wand des Körpers (2) Durchgangslöcher (9) befinden, und auf der äußeren Oberfläche des Körpers (2) ein Vorsprung mit einer Schraubenlinie vorhanden ist und ein Gewinde (4) bildet, wobei die Hülle der Schraubenlinie eine Geradlinie (5) ist, und die Hülle des Gewindes (4) auf einer Teillänge des Implantats (1) konvergiert zu einem Ende des Implantats (1) hin und die Form einer parametrischen B-Spline-Kurve (6) hat unter Beibehaltung der Tangentialität dieser Kurve zum Abschnitt der Geradlinie (5) der Hülle an ihrem Kontaktpunkt (7), und die Durchgangslöcher (9) in der Wand des Körpers (2) sind zwischen jeder Windung des Gewindes (4) am Boden (8) seiner Aussparung, **dadurch gekennzeichnet, dass** die Löcher (9) in der Wand des Körpers (2) einen Querschnitt haben, der in Richtung zum axialen Kanal (3) im Körper (2) konvergiert, insbesondere eine Kegelstumpfform aufweisen.

2. Implantat nach Anspruch 1, **dadurch gekennzeichnet, dass** sich die Hülle des Gewindes (4) zu einem Ende des Implantats (1) hin über eine Länge von 40-60 % der Gesamtlänge des Implantats (1) konvergiert, und bevorzugst über die Länge von 50 % der Gesamtlänge des Implantats (1).

3. Implantat nach Anspruch 1 oder 2, **dadurch gekennzeichnet, dass** auf einem Teil der Länge des Implantats (1) von seinem anderen Ende her mindestens zwei Längsschnitte (10) in der Wand des Körpers (2) eingebracht sind.

4. Implantat nach Anspruch 3, **dadurch gekennzeichnet, dass** die Längsschnitte (10) auf einer Länge von höchstens 60 % der Gesamtlänge des Implantats (1), besonders bevorzugt auf höchstens 50 % seiner Gesamtlänge, eingebracht sind.

## Revendications

1. Implant médical (1) en matériau biorésorbable, utilisé pour renforcer la pénétration des greffons dans la reconstruction des ligaments dans les tunnels osseux, dont le corps (2) se présente sous la forme d'un tube avec un canal axial longitudinal (3), la paroi du corps (2) est percée de trous (9), et sur la surface extérieure du corps (2) est placée une projection avec une ligne hélicoïdale formant un fil (4), l'enveloppe de la ligne hélicoïdale étant une ligne droite (5), et sur une partie de la longueur de l'implant (1), l'enveloppe du filet (4) converge vers une extrémité de l'implant (1) et se présente sous la forme d'une courbe paramétrique en vis B (6) tout en maintenant la tangence de cette courbe au segment de droite (5) de l'enveloppe à leur point de contact (7), tandis que les trous traversants (9) dans la paroi du corps (2) sont situés entre chaque bobine de fil (4) au fond (8) de sa feuillure, **caractérisés par le fait que les** trous (9) dans la paroi du corps (2) ont une forme de section transversale convergente dans la direction du canal axial (3) dans le corps (2), en particulier, la forme d'un cône tronqué.

2. Implant médical, selon la revendication 1, est **caractérisé en ce que** l'enveloppe du fil (4) converge vers une extrémité de l'implant (1) sur une longueur de 40-60% de la longueur de l'ensemble de l'implant (1), de préférence sur une longueur de 50% de l'ensemble de l'implant (1).

3. Implant médical, selon les revendications 1 et 2, est **caractérisé en ce que** sur une partie de la longueur de l'implant (1) à partir de son autre extrémité dans la paroi du corps (2), au moins deux fentes longitudinales (10) sont pratiquées.

4. Implant médical, selon la revendication 3, est **caractérisé en ce que** les fentes (10) sont réalisées sur au plus 60% de la longueur de l'implant entier (1), de préférence au plus 50%.
